(19) **Europäisches Patentamt**

**European Patent Office**

**Office européen des brevets**

(11) **EP 1 175 522 B1**

(12) **EUROPÄISCHE PATENTSCHRIFT**

(45) Veröffentlichungstag und Bekanntmachung des Hinweises auf die Patenterteilung:
**18.02.2004 Patentblatt 2004/08**

(21) Anmeldenummer: **00920669.9**

(22) Anmeldetag: **06.04.2000**

(51) Int Cl.[7]: **D01F 6/70**

(86) Internationale Anmeldenummer:
**PCT/EP2000/003060**

(87) Internationale Veröffentlichungsnummer:
**WO 2000/063472 (26.10.2000 Gazette 2000/43)**

(54) **ELASTANSPULE**

ELASTANE PACKAGE

BOBINE D'ELASTANE

(84) Benannte Vertragsstaaten:
**IT**

(30) Priorität: **19.04.1999 DE 19917529**

(43) Veröffentlichungstag der Anmeldung:
**30.01.2002 Patentblatt 2002/05**

(73) Patentinhaber: **Bayer Faser GmbH**
**41538 Dormagen (DE)**

(72) Erfinder:
• **REINEHR, Ulrich**
**D-41539 Dormagen (DE)**
• **SEHM, Tilo**
**D-40591 Düsseldorf (DE)**
• **ANDERHEGGEN, Wolfgang**
**D-41539 Dormagen (DE)**
• **HERBERTZ, Toni**
**D-41540 Dormagen (DE)**

(56) Entgegenhaltungen:
**WO-A-00/00681** **WO-A-00/00682**

• **PATENT ABSTRACTS OF JAPAN vol. 1998, no. 11, 30. September 1998 (1998-09-30) & JP 10 152264 A (TOYOBO CO LTD), 9. Juni 1998 (1998-06-09)**
• **PATENT ABSTRACTS OF JAPAN vol. 1998, no. 08, 30. Juni 1998 (1998-06-30) & JP 10 072726 A (DU PONT TORAY CO LTD), 17. März 1998 (1998-03-17)**

**Beschreibung**

Die Erfindung betrifft eine Elastanspule mit einem Wickel von Elastanfäden mit einem Titer von größer 160 dtex, insbesondere von 160 dtex bis 1400 dtex und einem Spulengewicht der Elastanfäden von mindestens 1,5 kg, dadurch gekennzeichnet, daß die Elastanfäden einen bändchenförmigen Fadenquerschnitt aufweisen, wobei das Verhältnis von Breite der Elastanfäden zur Dicke der Elastanfäden im Querschnitt mindestens drei zu eins beträgt.

Die Elastanspule wird zur Herstellung von textilen Flächengebilden und zur Herstellung von Einweghygieneartikeln verwendet.

Elastanfäden im Sinne der Erfindung sind mono- oder multifile Endlosfäden, die zu mindestens 85 Gew.-% aus segmentierten Poly(urethan)en bzw. Poly(urethanharnstoff)en bestehen. Die fadenbildenden Polymere weisen eine Segmentstruktur auf, d. h. sie bestehen aus "kristallinen" und "amorphen" Blöcken ("Hartsegmente" und "Weichsegmente"). Die Hartsegmente wirken aufgrund ihrer "Kristallinität" als Fixpunkte des Netzwerks und sind damit maßgebend für die Festigkeit der aus den Polymeren hergestellten Formkörper oder Fasern. Die Weichsegmente hingegen, deren Glastemperatur unterhalb der Gebrauchstemperatur liegen muß, sind für die Elastizität der Elastane maßgebend.

Derartige Elastane werden üblicherweise durch Polyaddition von langkettigen Dihydroxylverbindungen (Makrodiole) mit Diisocyanaten und niedermolekularen Dihydroxyl- oder Diaminoverbindungen als Kettenverlängerem hergestellt. Für hochwertige Elastanfilamente (auch als Spandex bezeichnet) werden durch Kettenverlängerung mit Diaminen gewonnene Poly(urethanharnstoff)e verwendet, weil sie gegenüber den Diol-verlängerten Poly(urethan)en aufgrund einer größeren Zahl von Wasserstoff-brückenbindungen zwischen den Polymerketten einerseits einen hohen Hartsegment-schmelzpunkt sowie andererseits ausgezeichnete mechanisch-elastische Eigenschaften aufweisen. Die Elastanfasern werden üblicherweise durch Verspinnen von Lösungen dieser segmentierten Poly(urethanharnstoff)e in hochpolaren Lösemitteln wie Dimethylformamid und Dimethylacetamid mittels des sogenannten Trockenspinnverfahrens oder des Naßspinnverfahrens oder alternativ unter Verzicht auf ein Spinnlösungsmittel durch Verspinnen aus der Schmelze hergestellt. Nach dem Verspinnen und gegebenenfalls Präparieren werden die Elastanfäden üblicherweise zu Spulen aufgewickelt.

In der Regel betragen die Spulengewichte für Elastanfäden je nach Titer und Einsatzgebiet 0,5 bis 1,5 kg. Es gibt jedoch auch Einsatzgebiete, wo eine noch größere Spulenlauflänge von Elastanfäden wünschenswert ist. Dies ist immer dann der Fall, wenn man den Weiterverarbeitungsprozeß, bei dem die Elastanfäden eingesetzt werden, nicht zu oft unterbrechen möchte, was zu häufigeren Maschinenstillständen und somit zu weiteren unerwünschten Kosten führt.

Eines dieser erwähnten Einsatzgebiete ist zum Beispiel die Herstellung von Hygieneartikeln z.B. von Einwegwindeln, bei denen die elastischen Fäden in die saugfähiges Material enthaltenden Windelfolien eingearbeitet werden. Hierdurch erreicht man eine gute Anpassungsfähigkeit der Windeln an die entsprechende Körperform. In der Regel werden auf jeder Längsseite der Windelfolien 2 - 3 elastische Fäden eingearbeitet. Die Einarbeitung der Elastanfäden in den Windelfolien erfolgt in der Regel durch Verklebung mit Heißschmelzklebern, sogenannten "Hotmelts". Derartige Heißschmelzkleber, die zu Konstruktionsverklebungen von z.B. PE-, PP-Folien und anderen nicht gewebten Folien geeignet sind, bestehen in der Regel aus synthetischen Kautschuken auf Isopren-Styrolbasis, welche zur Verbesserung ihrer Fließfähigkeit mit Mineralölen und Zusatzstoffen versetzt werden.

Um eine gute Verklebung der Elastanfäden mit den Windelfolien zu erreichen, sollten die Elastanfäden gewöhnlich möglichst präparationsfrei bzw. präparationsarm hergestellt werden.

Im Stand der Technik sind bisher zwei Verfahren bekannt geworden, welche die Herstellung von Elastanspulen beschreiben. Im japanischen Patent JP 2.000.038 werden Elastanspulen mit mindestens 1,5 kg, Spulengewicht beschrieben, wobei der Präparationsgehalt der Elastanfäden auf den Spulen kleiner 2 Gewichtsprozent beträgt. Die beschriebenen Elastane umfassen den Titerbereich von 308 bis 1232 dtex, entsprechend etwa 280 bis 1120 den.

Im japanischen Patent JP 10.305.060 werden Elastanspulen von mehr als 0,8 kg beansprucht, welche über eine Fadenreserve auf einer anderen Spule hergestellt werden. Es werden dazu Elastanfäden im gleichen Titerbereich von 308 bis 1232 dtex mit einem Präparationsgehalt von 0,01 - 1 Gewichtsprozent auf Spulenhülsen gewickelt.

Diese Elastanspulenart ist wiederum in der Praxis, besonders bei einem hohen Spulengewicht sehr schwierig weiterzuverarbeiten, weil Elastanfäden die Neigung haben, untereinander stark zu haften. Aufgrund der hohen Spulengewichte drücken naturgemäß die Außenlagen der Fäden wegen ihrer Wickelspannung verstärkt auf die Innenlagen der Fäden, so daß die Fäden nach Herstellung und Lagerung nur noch schlecht von den Spulen ablaufen. Man beobachtet dabei den sogenannten "Schuhsohleneffekt". Das heißt, ganze Fadenschichten auf den Spulen verkleben und lassen sich nicht mehr abspulen, wenn man die Elastanfäden schon während ihrer Herstellung nicht ausreichend präpariert.

Andererseits darf, wie bereits erwähnt, der Präparationsgehalt der Elastanfäden auf den größeren Spulen auch nicht zu hoch sein, um eine gute Verklebung der Fäden mit den Windelfolien zu erzielen.

Es wurde nun überraschenderweise gefunden, daß man dieses Problem dadurch lösen kann, daß man bei den Elastanfäden von Fäden ausgeht, die eine ganz bestimmte Querschnittsform aufweisen, so daß auch bei gegebenenfalls relativ hohem Präparationsgehalt der Elastanfäden auf den Spulen von z.B. über 1 Gew. %, insbesondere auch über 2 Gew. %, eine gute Verklebung mit den Windelfolien möglich ist.

Gegenstand der Erfindung ist eine Elastanspule mit einem Wickel von Elastanfäden mit einem Titer von größer 160 dtex, insbesondere von 160 dtex bis 1400 dtex und einem Spulengewicht der Elastanfäden von mindestens 1,5 kg, dadurch gekennzeichnet, daß die Elastanfäden einen bändchenförmigen Fadenquerschnitt aufweisen, wobei das Verhältnis von Breite der Elastanfäden zur Dicke der Elastanfäden im Querschnitt mindestens drei zu eins, insbesondere mindestens fünf zu eins, besonders bevorzugt mindestens sieben zu eins, ganz besonders bevorzugt mindestens zehn zu eins beträgt.

Bevorzugt beträgt das Spulengewicht der Elastanspule von 1,5 bis 10 kg, insbesondere von 1,8 bis 9 kg, besonders bevorzugt von 3 bis 8 kg.

Wie bereits erwähnt, lassen sich Elastanfäden trotz deutlich höherem Präparationsgehalt, auf Spulen mit Spulengewichten von 1,5 bis 5 kg und mehr herstellen und anschließend bei der Einarbeitung in Windelfolien noch einwandfrei unter üblichen Produktionsbedingungen verkleben, wenn man von Elastanfäden einer bestimmten Querschnittsform ausgeht. Ein weiterer Vorteil der Erfindung ergibt sich daraus, daß man noch feinere Elastanfäden von zum Beispiel 150 dtex trotz ihrer geringeren Querschnittsflächen durch Aufsprühen des Klebemittels noch einwandfrei verkleben kann, weil sie nicht in der üblichen kompakten, runden Querschnittsform, sondern als Bändchenform mit einer größeren Bändchenbreite vorliegen.

Für die Herstellung der Elastane der Elastanspulen ist auch besonders Recyclingmaterial von Elastanen geeignet, welches noch erhebliche Anteile an ursprünglichen Präparationsmitteln aufweist.

Bevorzugt ist eine Elastanspule, bei der die Elastanfäden einen Gehalt an Präparationsmittel von größer 1 Gew.-%, insbesondere von größer 2 Gew.-%, insbesondere bevorzugt von größer 2,2 Gew.-%, im Fadenvolumen und/oder an der Fadenoberfläche verteilt enthalten.

Die Elastanfäden sind insbesondere mehrfädig und weisen insbesondere mehr als 80, bevorzugt mehr als 160, besonders bevorzugt mehr als 400 Elementarfäden auf.

Besonders bevorzugt ist eine Elastanspule die einen Wickel aus Elastanfäden aufweist, bei dem der Einzelspinntiter (EST) der Elementarfäden der Elastanfäden kleiner 15 dtex, vorzugsweise kleiner 10 dtex, ist.

Die Elastanfäden bestehen bevorzugt aus Polyurethanen oder Polyharnstoffurethanen auf Basis von Polyestern und/oder Polyethern.

Die Elastanfäden der Spule sind insbesondere nach dem Schmelzspinnverfahren, nach dem Trockenspinnverfahren oder nach dem Naßspinnverfahren hergestellt, besonders bevorzugt sind sie nach dem Naßspinnverfahren hergestellt.

Die Querschnittsform von Elastanfäden ist weitgehend vom Herstellverfahren abhängig. Während man beim Trockenspinnen von Elastanfäden für mittlere und grobe Titer ab ca. 160 dtex aus Düsen mit mehreren Düsenlöchern verklebte Multifilamente mit, über den gesamten Querschnitt gemittelt, meist rundlichen bis ovalen Gesamtquerschnittsformen erhält, werden beim Naßspinnprozeß ab mittleren Titern bändchenförmige Querschnitte in Mehrfachreihen, wie an einer Perlenschnur aufgereiht, erhalten. Wegen der breiten Querschnittsform kann man deshalb besonders gut Klebemittel auf den Fäden, trotz eines relativ hohen Ölgehaltes von über 2 Gew. %, auftragen und mit den Windelfolien verkleben.

Unter Präparationsmittel werden im Sinne der Erfindung alle Arten von Zusätzen verstanden, die den Elastanfäden zur Verbesserung ihrer Verarbeitbarkeit und zum Aufbau der Spule zugesetzt werden können, insbesondere Gleitmittel, Netzmittel, Antistatika, Fadenschlußmittel etc. Funktion und geeignete allgemeine Typen von Präparationsmittel werden in "Synthesefasern", Herausgeber: B. v. Falkai, Verlag Chemie, 1981, S. 111ff genannt. Besonders geeignete Präparationsmittel sind Mineralöle oder Paraffine, Polysiloxane, insbesondere Polydimethylsiloxan oder ethoxyliertes Polydimethylsiloxan, Metallsalze höherer Fettsäuren wie Palmitinsäure, Stearinsäure oder Ölsäure, insbesondere deren Lithium-, Magnesium-, Zink-, Aluminium- oder Kalziumsalze. Weiter geeignete Präparationsmittel sind Antistatika wie Sulfosuccinate, insbesondere Dialkylsulfosuccinate. Die Präparationsmittel können in beliebigen Kombinationen und Mischungen eingesetzt werden.

Die Präparationsmittel können sowohl dem Elastan oder der Elastanlösung vor dem Verspinnen zugesetzt als auch den Fäden nach dem Verspinnen aufgetragen sein z.B. mittels Präparationswalzen oder Sprühaggregaten.

Die Elastanfäden können eine Vielzahl von verschiedenen anderen Zusatzstoffen für verschiedene Zwecke enthalten, wie beispielsweise Antioxidantien, Stabilisatoren gegen Wärme, Licht und UV-Strahlung, Pigmente und Mattierungsmittel, Farbstoffe, Schmier- und Gleitmittel. Beispiele für Antioxidantien, Stabilisatoren gegen Wärme, Licht und UV-Strahlung sind Stabilisatoren aus der Gruppe der sterisch gehinderten Phenole, der HALS-Stabilisatoren (hindered amine light stabilizer), der Triazine, der Benzophenone und der Benzotriazole. Beispiele für Pigmente und Mattierungsmittel sind Titandioxid, Zinkoxid und Bariumsulfat. Beispiele für Farbstoffe sind saure Dispersions- und Pigmentfarbstoffe und optische Aufheller.

Wie in WO 00/00681 und WO 00//00682 erwähnt wird, kann man bei einem Naßspinnprozeß von Elastanfäden durch Erhöhung der Filamentzahl bei gleichbleibenden Endtiter die Bändchenform von bis zu ca. 40 % verbreitern, wenn der Einzelspinntiter der Einzelfäden kleiner 15 dtex, insbesondere höchstens 10 dtex beträgt. Elastanfäden, bei denen die Breite der Elastanfäden im Querschnitt mindestens das 3 - 5 fache der Bändchendicke beträgt, sind besonders geeignet als Spulenmatenal für z.B. die Herstellung von Einweghygieneartikeln.

Weiterer Gegenstand der Erfindung ist die Verwendung der erfindungsgemäßen Elastanspulen zur Herstellung von textilen Flächengebilden, insbesondere von Gestrick, Gewebe oder Gewirke und zur Herstellung von Einweghygieneartikeln, insbesondere von Windelartikeln, z.B. für die Bündchen von Einwegwindeln.

Die Angabe der Bändchenbreite in den Beispielen erfolgt in "Mikron" (µm). 1 cm entspricht jeweils 10.000 µm.

Der Einzelspinntiter (EST) errechnet sich wie folgt:

$$EST = \frac{F \cdot K \cdot 0.94 \cdot 100 \ (dtex)}{A \cdot Z}$$

hierbei bedeuten:

F die Fördermenge an Spinnlösung (cm$^3$/min.), K die Konzentration der Spinnlösung (Gew. %), A die Geschwindigkeit der Fäden im Fällbad (m/min) und Z die Anzahl der Düsenlöcher.

Der Einzelspinntiter ist eine Meßgröße, die das Fadengewicht unmittelbar nach Austritt der Spinnlösung in das Fällbad angibt.

Messung des Präparationsölgehaltes:

Feuchtebestimmung R (%):

Ca. 3 g Elastanfäden werden abgewogen, Gewicht = W1. Die Fäden werden 1 Stunde bei 105°C im Trockenschrank getrocknet, auf Raumtemperatur abkühlen gelassen und anschließend zurückgewogen, Gewicht = W2

$$\text{Der Feuchtegehalt R (\%)} = \frac{(W1 - W2) \times 100}{W2}$$

Bestimmung des Präparationsgehaltes D (%):

Die Fadenprobe wird in ein Becherglas gegeben und mit 100 ml reinem Petrolether benetzt. Anschließend wird 10 Minuten mit einem Rührer (Magnetrührer) bei Raumtemperatur stark durchgerührt. Dann wird die Probe aus dem Becherglas genommen und nochmals mit 100 ml frischem Petrolether 10 Minuten lang stark gerührt. Der Petrolether wird abgegossen und die Fäden an der Luft getrocknet. Anschließend werden die Fäden 5 Minuten in 100 ml kochendem Wasser behandelt und 1 Stunde im Trockenschrank bei 105°C getrocknet, auf Raumtemperatur abkühlen gelassen und ausgewogen, Gewicht = W 4.

Der Präparationsgehalt D berechnet sich dann wie folgt:

$$D \ (\text{in \%}) = \left[ \frac{1 - \dfrac{W4}{W2}}{1 + \dfrac{R}{100}} \right] \cdot 100$$

Die folgenden Beispiele dienen der näheren Erläuterung der Erfindung. Teile und Prozentangaben beziehen sich, falls nicht anders vermerkt, auf das Gewicht.

**Beispiele:**

**Beispiel 1**

Eine 30 gew.%ige Elastan-Spinnlösung hergestellt entsprechend Beispiel 2 aus der DE-054222772, die mit 0,5 % Diethylamin ca. 12 min. bei 130°C vorbehandelt wurde und eine Spinnviskosität von 23 Pa.s, gemessen bei 50°C aufwies, wurde aus vier 172-Lochdüsen mit 0,1 mm Bohrungsdurchmesser in ein Fällbad aus 15 %iger DMAC-Wasserlösung gesponnen. Die Fäden wurden über eine Umlenkrolle, die knapp oberhalb der Fällbadflüssigkeit liegt, mit 60 m/min abgezogen, koalesziert, gewaschen und mit 125 m/min. Geschwindigkeit über eine Heizrolle fixiert, präpariert und die verzwimten Fäden auf einen Wickler aufgespult. Die Fäden wurden auf Hülsen von 115 mm Breite aufgenommen. Es wurden 8 Spulen mit einem Spulengewicht von 5 kg hergestellt. Aus der Spinnpumpe, welche die vier Düsen versorgte, wurden 106,5 cm$^3$/min Spinnlösung gefördert. Der Einzelspinntiter (EST) lag bei 7,3 dtex. Der Elastanfaden, der einen Endtiter von 603 dtex aufweist, liegt in Form eines geschlossenen Bändchens vor. Die Bändchenbreite beträgt ca. 1180 µm und die Bändchendicke liegt bei ca. 190 µm. Das Verhältnis Bändchenbreite zu Bändchendicke beträgt somit 6,2 zu 1. Die Elastanfäden hatten einen Feuchtegehalt R von 0,17 % und einen Präparationsgehalt von 2,23 %. 8 Stück Elastanspulen vom Spulengewicht 5 kg wurden an einer Produktionsstraße für Einwegwindeln auf einen Abwikkelstand gelegt und unter Produktionsbedingungen mit 160 m/min mit den vorgelegten Windelfolien durch Aufsprühung von Heißschmelzkleber (Hotmelts; Lunatack D3937, Hersteller H.B. Fuller GmbH) verklebt. Die fertigen Windeln zeigten eine sehr gute Haftung zwischen den eingearbeiteten Elastanfäden und den Windelfolien

(Bundprüfungen im Blaulicht).

### Beispiel 2

Ein Teil der Spinnlösung aus Beispiel 1 wurde aus vier 96-Lochdüsen mit 0,1 mm Bohrungsdurchmesser in einem Fällbad aus 15 %iger DMAC-Wasserlösung gesponnen. Die Fäden wurden mit 90 m/min abgezogen, koalesziert, gewaschen und mit 150 m/min Geschwindigkeit über eine Heizrolle fixiert, präpariert und die verzwimten Fäden auf einen Wickler aufgespult. Die Fäden wurden auf Hülsen von 115 mm Hülsenbreite aufgenommen. Es wurden 8 Spulen mit einem Spulengewicht von 1,5 kg hergestellt. Aus der Spinnpumpe, welche die vier Düsen versorgte, wurden 31,6 ccm/min Spinnlösung gefördert. Der Einzelspinntiter (EST) lag bei 2,6 dtex. Der Elastanfaden, der einen Endtiter von 149 dtex aufweist, liegt wieder in Form eines geschlossenen Bändchens vor. Die Bändchenbreite beträgt ca. 450 µm und die Bändchendicke liegt bei ca. 65 µm. Das Verhältnis Bändchenbreite zu Bändchendicke beträgt 7 zu 1. Die Elastanfäden hatten einen Feuchtegehalt R von 0,14 % und einen Präparationsgehalt von 2,11 %. 8 Stück Elastanspulen vom Gewicht 1,5 kg wurden an einer Produktionsstraße für Einwegwindeln auf einen Umwickelstand gelegt und wie in Beispiel 1 beschrieben, mit Windelfolien zu fertigen Windeln verarbeitet. Die fertigen Windeln zeigten wiederum eine gute Haftung zwischen den eingearbeiteten Elastanfäden und den Windelfolien.

### Beispiel 3

Eine 30 gew.%ige Elastan-Spinnlösung hergestellt nach Beispiel 1, wurde hergestellt aus Recyclingmaterial gemäß Beispiel 1 der Anmeldung FAS 13, im Verhältnis 1:1 vermischt und wie dort beschrieben in eine Spinnlösung mit einer Spinnviskosität von 18 Pa.s., gemessen bei 50°C überführt. Die Spinnlösung wurde aus vier 172-Lochdüsen mit 0,15 mm Bohrungsdurchmesser in ein Fällbad aus 20 %iger DMAC-Wasserlösung gesponnen. Die Fäden wurden anschließend mit 70 m/min abgezogen, koalesziert, gewaschen und mit 130 m/min Geschwindigkeit über eine Heizrolle fixiert, präpariert und die verzwirnten Fäden wiederum auf einen Wickler aufgespult. Die Fäden wurden auf Hülsen von 115 mm Breite aufgenommen. Es wurden 8 Spulen mit einem Spulengewicht von 3 kg hergestellt. Aus der Spinnpumpe, welche die 4 Düsen versorgte, wurden 110,8 cm³/min Spinnlösung gefördert. Der Einzelspinntiter (EST) lag bei 6,5 dtex. Der Elastanfaden, der einen Endtiter von 598 dtex aufweist, liegt wiederum in Form eines geschlossenen Bändchens vor. Die Bändchenbreite beträgt ca. 1100 µm und die Bändchendicke liegt bei 200 µm. Das Verhältnis Bändchenbreite zu Bändchendicke beträgt somit 5,5 zu 1. Die Elastanfäden hatten einen Feuchtegehalt R von 0,17 % und einen Präparationsgehalt D von 4,29 %, verteilt über den gesamten Fadenquerschnitt. 8 Stück Elastanspulen vom Spulengewicht 3 kg wurden wie in Beispiel 1 beschrieben einer Produktionsstraße für Einwegwindeln vorgelegt und in die Windelfolien eingearbeitet. Die fertigen Windeln zeigten eine sehr gute Haftung zwischen den eingearbeiteten Elastanfäden und den Windelfolien.

### Beispiel 4

Ein Teil der Spinnlösung aus Beispiel 3 wurde aus vier 96-Lochdüsen mit 0,2 mm Bohrungsdurchmesser, wie in Beispiel 3 beschrieben, in ein Fällbad aus 10 %iger DMACWasserlösung gesponnen. Die Fäden wurden anschließend mit 80 m/min abgezogen, koalesziert, gewaschen und mit 125 m/min Geschwindigkeit über eine Heizrolle fixiert, präpariert und auf einen Wickler aufgespult. Es wurden wieder 8 Spulen mit einem Spulengewicht von 3 kg hergestellt. Aus der Spinnpumpe, welche die 4 Düsen versorgte, wurden 142,7 cm³/min Spinnlösung gefördert. Der Einzelspinntiter (EST) lag bei 13,1 dtex. Der Elastanfaden, der einen Endtiter von 805 dtex aufweist, liegt wiederum als geschlossene Bändchenform vor. Die Bändchenbreite beträgt ca. 760 µm und die Bändchendicke liegt bei ca. 250 µm. Das Verhältnis Bändchenbreite zu Bändchendicke beträgt somit ca. 3 zu 1. Die Elastanfäden hatten einen Feuchtegehalt R von 0,2 % und einen Präparationsgehalt D von 3,88 %, verteilt über den samten Fadenquerschnitt. 8 Stück Elastanspulen vom Spulengewicht 3 kg wurden wiederum wie in Beispiel 1 beschrieben einer Produktionsstraße für Einwegwindeln vorgelegt und in Windelfolien eingearbeitet. Wie Bundprüfungen an Windeln unter Blaulichtbestrahlung zeigten, liegt wiederum eine sehr gute Haftung zwischen den Elastanfäden und den Windelfolien vor.

### Beispiel 5

Eine 30 %ige Spinnlösung, hergestellt aus reinem Recyclingmaterial, gemäß Beispiel 2 der Anmeldung FAS 13, mit einer Spinnviskosität von 22 Pa.s gemessen bei 50°C, wurde aus zwei 441 Lochdüsen mit 0,12 mm Bohrungsdurchmesser in ein Fällbad aus 18 %iger DMAC-Wasserlösung gesponnen. Die Fäden wurden mit 60 m/min abgezogen, koalesziert, gewaschen und mit 130 m/min über eine Heizrolle fixiert, präpariert und auf einen Wickler aufgespult. Es wurden 8 Spulen mit einem Spulengewicht von 2,4 kg hergestellt. Aus der Spinnpumpe, welche die zwei Düsen versorgte, wurden 129 cm³/min. Spinnlösung gefördert. Der Einzelspinntiter (EST) lag bei 6,9 dtex. Der Elastanfaden, der einen Endtiter von 1402 dtex aufweist, liegt wiederum als geschlossene Bändchenform vor. Die Bändchenbreite beträgt 3330 µm und die Bändchendicke liegt bei ca. 320 µm. Das Verhältnis Bändchenbreite zu Bändchendicke beträgt somit 10,4 : 1. Die Elastanfäden hatten einen Feuchtegehalt R von 0,2 % und einen Präparationsgehalt D von 2,67 %, verteilt über den gesamten Fadenquerschnitt. 8 Stück Elastanspulen vom Spulengewicht 2,4 kg wurden wiederum einer Produktionsstraße für Einwegwindeln vorgelegt und in Windelfolien eingearbeitet. Wie Bundprüfungen an Windeln unter Blaulichtbestrahlung zeigten, liegt wiederum eine sehr gute Haftung zwischen den Elastanfäden und den Windelfolien vor.

## Patentansprüche

1. Elastanspule mit einem Wickel von Elastanfäden mit einem Titer von größer 160 dtex, insbesondere von 160 dtex bis 1400 dtex und einem Spulengewicht der Elastanfäden von mindestens 1,5 kg, **dadurch gekennzeichnet, daß** die Elastanfäden einen bändchenförmigen Fadenquerschnitt aufweisen, wobei das Verhältnis von Breite der Elastanfäden zur Dicke der Elastanfäden im Querschnitt mindestens drei zu eins, insbesondere mindestens fünf zu eins, besonders bevorzugt mindestens sieben zu eins, ganz besonders bevorzugt mindestens zehn zu eins beträgt.

2. Elastanspule nach Anspruch 1, **dadurch gekennzeichnet, daß** das Spulengewicht von 1,5 bis 10 kg, insbesondere von 1,8 bis 9 kg, besonders bevorzugt von 3 bis 8 kg beträgt.

3. Elastanspule nach Anspruch 1 oder 2, **dadurch gekennzeichnet, daß** die Elastanfäden einen Gehalt an Präparationsmittel von größer 1 Gew.-%, insbesondere von größer 2 Gew.-%, insbesondere bevorzugt von größer 2,2 Gew.-%, im Fadenvolumen und/oder an der Fadenoberfläche verteilt enthalten.

4. Elastanspule nach einem der Ansprüche 1 bis 3, **dadurch gekennzeichnet, daß** die Elastanfäden mehrfädig sind und insbesondere mehr als 80, bevorzugt mehr als 160, besonders bevorzugt mehr als 400 Elementarfäden aufweisen.

5. Elastanspule nach Anspruch 1, **dadurch gekennzeichnet, daß** der Einzelspinntiter (EST) der Elementarfäden Elastanfäden kleiner 15 dtex, vorzugsweise kleiner 10 dtex, ist.

6. Elastanspule nach einem der Ansprüche 1 bis 5, **dadurch gekennzeichnet, daß** die Elastanfäden aus Polyurethanen oder Polyharnstoffurethanen auf Basis von Polyestern und/oder Polyethern bestehen.

7. Elastanspule nach einem der Ansprüche 1 bis 6, **dadurch gekennzeichnet, daß** die Elastanfäden nach dem Schmelzspinnverfahren, nach dem Trockenspinnverfahren oder nach dem Naßspinnverfahren hergestellt sind, insbesondere nach dem Naßspinnverfahren

8. Verwendung der Elastanspulen nach einem der Ansprüche 1 bis 7 zur Herstellung von textilen Flächengebilden, insbesondere Gestrick, Gewebe oder Gewirke und zur Herstellung von Einweghygieneartikeln, insbesondere von Windelartikeln.

## Claims

1. Elastane package comprising a reel of elastane threads having a titre greater than 160 dtex, in particular of 160 dtex to 1400 dtex and a package weight of the elastane threads of at least 1.5 kg, **characterized in that** the elastane threads have a ribbon-shaped thread cross section, wherein the ratio of width of the elastane threads to thickness of the elastane threads in cross section is at least three to one, in particular at least five to one, particularly preferably at least seven to one, most particularly preferably at least ten to one.

2. Elastane package according to claim 1, **characterized in that** the package weight ranges from 1.5 to 10 kg, in particular from 1.8 to 9 kg, particularly preferably from 3 to 8 kg.

3. Elastane package according to claim 1 or 2, **characterized in that** the elastane threads have a conditioning agent content of more than 1 wt.%, in particular of more than 2 wt.%, particularly preferably of more than 2.2 wt.%, which is distributed in the thread volume and/or on the thread surface.

4. Elastane package according to one of claims 1 to 3, **characterized in that** the elastane threads are multifilament and comprise in particular more than 80, preferably more than 160, particularly preferably more than 400 elementary filaments.

5. Elastane package according to claim 1, **characterized in that** the individual spinning titre (IST) of the elementary filaments of the elastane threads is lower than 15 dtex, preferably lower than 10 dtex.

6. Elastane package according to one of claims 1 to 5, **characterized in that** the elastane threads are made from polyester- and/or polyether-based polyurethanes or polyurea urethanes.

7. Elastane package according to one of claims 1 to 6, **characterized in that** the elastane threads are manufactured using the melt spinning process, using the dry spinning process or using the wet spinning process, in particular using the wet spinning process.

**8.** Use of the elastane packages according to one of claims 1 to 7 for manufacturing flat textile materials, in particular knitted or woven fabrics and for manufacturing disposable hygiene articles, in particular nappy articles.

**Revendications**

**1.** Bobine d'élasthanne dotée d'un rouleau de fils d'élasthanne avec un titre d'une grosseur de 160 dtex, tout particulièrement de 160 dtex à 1400 dtex et un poids de bobine de fils d'élasthanne d'au moins 1,5 kg, **caractérisée en ce que** les fils d'élasthanne présentent une section de fil en forme de petit ruban, moyennant quoi le rapport de la largeur des fils d'élasthanne sur la grosseur des fils d'élasthanne est en section transversale d'au moins trois sur un, particulièrement d'au moins cinq sur un, de manière particulièrement préférée d'au moins sept sur un, et de manière tout particulièrement préférée d'au moins dix sur un.

**2.** Bobine d'élasthanne selon la revendication 1, **caractérisée en ce que** le poids de la bobine se situe entre 1,8 et 9 kg, de manière particulièrement préférée entre 3 et 8 kg.

**3.** Bobine d'élasthanne selon la revendication 1 ou 2, **caractérisée en ce que** les fils d'élasthanne ont une teneur en moyen de préparation supérieure à 1% en poids, particulièrement supérieure 2% en poids, de manière particulièrement préférée supérieure à 2,2% en poids, répartie dans le volume de fils et/ou au niveau de la surface des fils.

**4.** Bobine d'élasthanne selon l'une des revendications 1 à 3, **caractérisée en ce que** les fils d'élasthanne ont plusieurs fils et présentent particulièrement plus de 80, de manière préférée plus de 160, de manière particulièrement préférée plus de 400 fils élémentaires.

**5.** Bobine d'élasthanne selon la revendication 1, **caractérisée en ce que** le titre de bobine unique (EST) des fils élémentaires de fils d'élasthanne est inférieur à 15 dtex, de préférence inférieur à 10 dtex.

**6.** Bobine d'élasthanne selon l'une des revendications 1 à 5, **caractérisée en ce que** les fils d'élasthanne sont constitués de polyuréthanes ou de polycarbamide uréthanes sur la base de polyesters et/ou de polyéthers.

**7.** Bobine d'élasthanne selon l'une des revendications 1 à 6, **caractérisée en ce que** les fils d'élasthanne sont fabriqués selon le processus de filage à chaud, selon le processus de filage à sec, ou selon le processus de filage à eau chaude, particulièrement selon le processus de filage à eau chaude.

**8.** Utilisation des bobines d'élasthanne selon l'une des revendications 1 à 7 afin de fabriquer des produits de surface textiles, tout particulièrement des tricots, des tissus ou des maillages et pour fabriquer des articles hygiéniques à usage unique, tout particulièrement des couches.